# EUROPÄISCHE PATENTSCHRIFT

(11) **EP 1 191 891 B1**
(45) Veröffentlichungstag und Bekanntmachung des Hinweises auf die Patenterteilung: **18.06.2003**
(21) Anmeldenummer: 99926227.2
(22) Anmeldetag: 07.07.1999
(51) Int. Cl.: A61B 17/86

(54) **KNOCHENSCHRAUBE MIT AXIAL ZWEISTÜCKIGEM SCHRAUBENFKOPF**
BONE SCREW WITH AXIALLY TWO-PART SCREW HEAD
VIS A OS AVEC TETE DE VIS CONSTITUEES AXIALEMENT DE DEUX PARTIES

(43) Veröffentlichungstag der Anmeldung: 03.04.2002
(73) Patentinhaber: SYNTHES AG Chur, 7002 Chur (CH)
(72) Erfinder: FRIGG, Robert, CH-2544 Bettlach (CH); FERUS, Robert, CH-2544 Bettlach (CH)
(74) Vertreter: Lusuardi, Werther Giovanni, Dr.
(86) Internationale Anmeldenummer: CH9900303
(87) Internationale Veröffentlichungsnummer: WO01003593

(56) Entgegenhaltungen:
- EP-A- 0 498 709
- EP-A- 0 507 162
- EP-A- 0 861 636
- US-A- 4 946 458
- US-A- 5 466 237
- US-A- 5 569 247

## Beschreibung

Die Erfindung bezieht sich auf eine Knochenschraube zur osteosynthetischen Knochenfixation gemäss dem Oberbegriff des Patentanspruchs 1.

Eine Knochenschraube gemäß dem Oberbegriff des Anspruchs 1 ist aus EP-A- 0 507 162 bekannt.

Aus dem Stand der Technik sind bereits verschiedene Vorrichtung zur internen Fixation von Knochenfragmenten im menschlichen oder tierischen Körper bekannt.

Im Falle der internen Fixation der Wirbelsäule oder von Wirbelsäulenteilen bestehen solche Vorrichtungen oft im wesentlichen aus Pedikelschrauben, welche in den Pedikeln der einzelnen zu verbindenden Wirbelkörper mittels Gewinden verankert werden, und einem oder mehreren sich in Richtung der Wirbelsäule erstreckenden Längsträger, welcher fest mit der Pedikelschraube verbindbar sein muss. Zur stabilen Verankerung des gesamten lmplantates müssen die Pedikelschrauben einerseits fest in die Pedikel eingeschraubt und andererseits starr mit den Längsträgern verbunden werden. Die Verbindung zwischen dem Schraubenkopf der Pedikelschrauben und dem Längsträger erfolgt üblicherweise mittels Klemmmechanismen, welche auch bei verschiedenen Winkeln der Pedikelschraube gegenüber dem Längsträger eine stabile Verbindung ermöglichen müssen. Die Klemmverbindung muss lösbar sein, damit das gesamte lmplantat ohne grosse Gewebeöffnungen im Bereich der Wirbelsäule wieder entfernbar ist.

Feste Verbindungen zwischen Knochenschrauben und Platten oder Trägern sind auch bei anderen internen Knochenfixationen häufig. Auch hier müssen verschiedene Abwinklung der Knochenschrauben gegenüber der Platte oder dem Träger möglich sein, ohne dass die Verbindungen in ihrer Stabilität beeinträchtigt werden.

Eine solche Verbindung zwischen einer Knochenverankerungsschraube and einem Stabilisationsstab zur internen Fixation von Wirbelkörpern ist aus der US 5,466,237 BYRD bekannt. Diese bekannte Erfindung weist eine Knochenverankerungsschraube mit einem Schraubenkopf auf, welcher an seiner dem Schraubenschaft zugewandten Seite kugelschichtförmig gestaltet ist und endständig konvex ausgebildet ist. Der kugelschichtförmige Teil der Schraube ist in einer Bohrung des Verankerungselementes gelagert, wobei diese Bohrung einen konkaven sich gegen den Schraubenschaft verjüngenden Abschnitt umfasst, so dass sich eine kugelgelenkartige Verbindung zwischen der Knochenschraube und dem Verankerungselement ergibt. Blockiert wird diese kugelgelenkartige Verbindung durch Anziehen einer Mutter am Verankerungselement, welche auf den in das Verankerungselement eingelegten Längsträger presst, welcher in der Folge auf den endständigen, konvexen Teil des Schraubenkopfes drückt und somit den Schraubenkopf im Verankerungselement blockiert.

Im Falle eines endständig konvexen Schraubenkopfes besteht die Gefahr, dass die Mittel zum Einführen eines Schraubendrehers im Schraubenkopf, beispielsweise ein zentral angeordneter Innensechskant, die Kontaktfläche zwischen einem auf den Schraubenkopf drückenden Längsträger und dem Schraubenkopf beeinträchtigen.

Hier will die Erfindung Abhilfe schaffen. Der Erfindung liegt die Aufgabe zugrunde, eine Knochenschraube mit zweistückigem Schraubenkopf herzustellen, wobei das Unterteil des Schraubenkopfes fest mit dem Schraubenschaft verbunden ist und das Oberteil des Schraubenkopfes nach der Implantation der Knochenschraube mit dem Unterteil verbindbar ist. Auf diese Weise wird ermöglicht, dass die Oberfläche eines konvexen Schraubenkopfes im Bereich der Berührung mit einem andern Implantatteil, beispielsweise einem Längsträger glatt ausführbar ist, wodurch sich ein Punktkontakt zwischen dem Oberteil des Schraubenkopfes mit dem Längsträger herstellen lässt. Mittels der erfindungsgemässen Vorrichtung ist eine Verbindung zwischen Knochenschraube und Verankerungselement herzustellbar, welche verschiedene Winkel zwischen Schraubenachse und Verankerungselement zulässt und stabil ist

Die Erfindung läst die gestellte Aufgabe mit einer Knochenschraube zur osteosynthetischen Knochenfixation, welche die Merkmale des Anspruchs 1 aufweist.

Weitere vorteilhafte Ausgestaltungen der Erfindung sind in den abhängigen Ansprüchen gekennzeichnet.

Die erfindungsgemässe Knochenschraube umfasst einen zu einer Längsachse konzentrisch in einem Knochen oder Knochenteil zu verankernden Schraubenschaft und einen ebenfalls konzentrischen, axial zweistückigen Schraubenkopf. Das schraubenschaftseitige Unterteil des Schraubenkopfes weist einen Durchmesser auf, der grösser ist als der Durchmesser des Schraubenschaftes. Das Unterteil ist schraubenschaftseitig kugelschichlförmig ausgestaltet. Das Oberteil des Schraubenkopfes ist auf das Unterteil aufsteckbar oder mit Unterteil und Schraubenschaft mittels eines Presssitzes, einer Konusverbindung, einer Schraubverbindung oder einer Bajonettverschlussverbindung verbindbar.

In der bevorzugten Ausführungsform der erfindungsgemässen Knochenschraube sind das Unterteil des Schraubenkopfes und der Schraubenschaft einstückig. Das Unterteil kann in anderen Ausführungsformen jedoch auch als separates Teil ausgeführt sein, beispielsweise als kreisringförmige Scheibe, welche über einen entsprechenden Zapfen am Schraubenschaft schiebbar ist und beim Befestigen des Oberteils des Schraubenkopfes zwischen diesem und dem Schraubenschaft festgeklemmt wird.

Der Durchmesser des Unterteils des Schraubenkopfes beträgt vorzugsweise zwischen 8 und 10 mm, während der Durchmesser des Schraubenschaftes vorzugsweise zwischen 5 und 6 mm beträgt.

In einer weiteren Ausführungsform der erfindungsgemässen Knochenschraube ist das Unterteil des zweistückigen Schraubenkopfes als kreisscheibenförmig ausgestaltet. Die Dicke eines solchen kreisscheibenförmig ausgebildeten Unterteils liegt vorzugsweise zwischen 1 und 2 mm. Vorzugsweise ist der Rand eines solchen Unterteils kantig ausgebildet und weist eine untere Kante zur Anlage an die Wand einer mit einer gekrümmten Fläche abgesetzten Bohrung auf. Dadurch lässt sich ein linienförmiger Kontakt erreichen.

Das Oberteil des Schraubenkopfes kann endständig konvex, insbesondere sphärisch und halbkugelförmig ausgebildet sein.

Die erfindungsgemässe Knochenschraube lässt sich je nach Ausführungsform zur Fixation von Knochen oder Knochenteilen innerhalb einer osteosynthetischen Fixationsvorrichtung einsetzen und kann beispielsweise zur Fixation von Knochen oder Knochenteilen an einer Knochenplatte oder auch zur Fixation von Wirbelkörpern innerhalb einer Wirbelsäulenfixationsvorrichtung dienen.

Die erfindungsgemässe Vorrichtung zur osteosynthetischen Knochenfixation umfasst mindestens eine Knochenschraube mit einem im Knochen oder Knochenteil zu verankernden Schraubenschaft und einem Schraubenkopf und mindestens einen Fixationskörper, welcher zur stabilen Fixation der Knochen oder Knochenteite dient. Der Fixationskörper weist mindestens eine Bohrung zur Aufnahme der Knochenschraube auf, wobei diese Bohrung den Fixationskörper durchdringt und einen konkaven sich gegen das schraubenschaftseitige Ende verjüngenden Abschnitt umfasst. Das Unterteil des Schraubenkopfes ist kugelschichtförmig oder kreisscheibenförmig, wobei der Durchmesser so dimensioniert ist, dass das Unterteil im konkaven Abschnitt der Bohrung unter verschiedenen Winkeln zwischen Längsachse der Knochenschraube und Zentralachse der Bohrung an der Wand der Bohrung auflegbar ist. Die Konfiguration mit einem scheibenförmigen Unterteil mit einer ebenen schraubenschaftseitigen Fläche, welche beim Anziehen der Schraube auf der konkaven Wand der Bohrung anliegt, ermöglicht einen linienförmigen Kontakt zwischen der Knochenschraube und dem Fixationskörper.

In einer Ausführungsform der erfindungsgemässen Vorrichtung client diese zur Verbindung eines Längsträgers mit der als Pedikelschraube ausgeführten Knochenschraube innerhalb eines Wirbelsäulenfixationssystems. Der Fixationskörper ist als Aufnahmekopf ausgeführt, welcher zur Verbindung des Längsträgers mit der Pedikelschraube dient. Neben der den Aufnahmekopf durchdringenden Durchgangsbohrung zur Aufnahme der Pedikelschraube ist zusätzlich ein quer zur Zentralachse des Aufnahmekopfes verlaufender, gegen das schraubenkopfseitige Ende offener Kanal zur Aufnahme des Längsträgers angebracht. Zudem umfasst die Vorrichtung zusätzlich Spannmittel, welche am schraubenkopfseitigen Ende mit dem Aufnahmekopf in lösbarer Weise verbindbar sind und zur Fixierung des Längsträgers und der Pedikelschraube innerhalb des Aufnahmekopfes dienen. Die Durchgangsbohrung umfasst einen konkaven sich gegen ihr schraubenschaftseitiges Ende verjüngenden Abschnitt, so dass der Schraubenkopf der Pedikelschraube im konkaven Abschnitt der Durchgangsbohrung unter verschiedenen Winkeln zwischen Schraubenachse und Zentralachse der Bohrung an der Wand der Durchgangsbohrung zur Anlage bringbar ist.

In einer weiteren Ausführungsform der erfindungsgemässen Vorrichtung weist das Unterteil des Schraubenkopfes einen Durchmesser d auf und der konkave Abschnitt ist sphärisch ausgebildet und weist einen Durchmesser D auf, wobei D = d ist. Durch diese Ausgestaltung sind jedoch bei einem kreisscheibenförmigen Unterteil nur kleine Winkel der Schraubenachse gegenüber der Zentralachse der Bohrung im Fixationskörper möglich, da sonst der linienförmige Kontakt nur auf einem Teil des Umfanges gegeben ist. Für grössere Winkel bei kreisscheibenförmigen Unterteilen eignet sich eine Ausgestaltung des konkaven Abschnittes mit einem Durchmesser D, wobei D > d ist. In diesem Fall lässt sich das Verhältnis d : D zwischen 0,5 und 1,0, vorteilhafterweise zwischen 0,85 und 0,95 wählen. Zudem ist der Durchmesser des Schraubenkopfes so gewählt, dass dieser bei Schrägstellung der Knochenschraube nicht an der Wand der Bohrung anliegt und dadurch eine Schrägstellung der Knochenschraube einschränken würde.

In wiederum einer anderen Ausführungsform der erfindungsgemässen Vorrichtung ist der konkave Abschnitt kugelschichtartig ausgebildet ist, wobei die Kugelschicht einen Radius X aufweist während der Durchmesser des konkaven Abschnittes D ist, so dass X ≥ D ist. Das Verhältnis von D/2 zu X beträgt zwischen 0,5 und 1,0, vorteilhafterweise zwischen 0,85 und 0,95.

Das endständig konvexe Oberteil des Schraubenkopfes ist in einer speziellen Ausführungsform der erfindungsgemässen Vorrichtung sphärisch oder halbkugelförmig aufgebildet ist. Dabei kann des Zentrum des sphärischen Oberteils mit dem Kugelzentrum der Kugelschicht am Unterteil zusammenfallen. Der Vorteil dieser Ausführung liegt im Fall der Verbindungsvorrichtung zwischen Längsträger und Pedikelschraube darin, dass ein zwischen Schraubenkopf und Spannmittel eingespannter Längsträger auch bei einer Schrägstellung der Pedikelschraube konzentrisch zur Zentralachse auf den Schraubenkopf drückt.

Der konvexe Schraubenkopf ist axial zweistückig, wobei das endständig Oberteil des Schraubenkopfes lösbar mit dem Unterteil, welches mit dem Schraubenschaft einstückig ist, verbunden sein kann. Durch die zweistückige Ausführung lassen sich vor allem die Mittel zum Einführen eines Schraubendrehers im Schraubenschaft, wie beispielsweise ein Innensechskant oder Innengewinde, einfacher anordnen. Zudem wird bei einer zentralen Anordnung beispielsweise eines Innensechskantes im Unterteil die Auflage zwischen Längsträger und Schraubenkopf durch Anbringen des Oberteils nach der Implantation der Knochenschraube nicht beeinträchtigt.

Im Falle eines kreisscheibenförmigen Unterteils wird der Rand des Unterteils vorteilhafterweise vor allem auf der schraubenschaftseitigen Unterseite kantig ausgebildet, so dass eine untere Kante entsteht, welche zu einem linienförmigen Kontakt mit der Wand des konkaven Abschnittes bestimmt ist.

Der Durchmesser D des konkaven Abschnittes und der Durchmesser d des Unterteils betragen vorteilhafterweise zwischen 8-10 mm. Im Falle eines kreisscheibenförmigen Unterteils weist dieses vorteilhafterweise eine Dicke von 1-2 mm auf.

Der Aussendurchmesser des Schraubenschaftes beträgt vorteilhafterweis 5-6 mm.

Die durch die Erfindung erreichten Vorteile sind im wesentlichen darin zu sehen, dass dank der erfindungsgemässen Gestaltung der Knochenschraube die Oberfläche des konvexen Schraubenkopfes im Bereich der Berührung mit einem anderen Implantatteil, beispielsweise dem Längsträger glatt ist und diese Kontaktzone nicht durch Mittel zur Aufnahme eines Schraubendrehers beeinträchtigt wird. Die Vorteile der erfindungsgemässen Vorrichtung liegen in der Gestaltung der Bohrung zur Aufnahme einer Knochenschraube und der Gestaltung der Knochenschraube mit einem Schraubenkopfunterteil, welches zur Auflage in einem konkaven Abschnitt der Bohrung bestimmt ist, so dass bei einem kreisscheibenförmig ausgestalteten Unterteil ein linienförmiger Kontakt herstellbar ist, welcher bei der Fixation der Vorrichtung zu einer festen Verbindung zwischen Knochenschraube und Fixationskörper führt. Im Falle einer deformierbaren Bohrungswand lässt sich wegen des linienförmigen Kontakts auch eine formschlüssige Verbindung zwischen Unterteil und Bohrungswand herstellen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht einer Ausführungsform der erfindungsgemässen mehrteiligen Knochenschraube;
Fig. 2 eine Ansicht einer weiteren Ausführungsform der erfindungsgemässen mehrteiligen Knochenschraube;
Fig. 3 eine Ansicht nochmals einer weiteren Ausführungsform der erfindungsgemässen mehrteiligen Knochenschraube;
Fig. 4 eine Ansicht nochmals einer weiteren Ausführungsform der erfindungsgemässen mehrteiligen Knochenschraube;
Fig. 5 einen Schnitt parallel zu einem Längsträger einer Ausführungsform der erfindungsgemässen Vorrichtung; und
Fig. 6 einen Schnitt durch die in Fig. 5 dargestellte Ausführungsform der erfindungsgemässen Vorrichtung quer zu einem Längsträger.

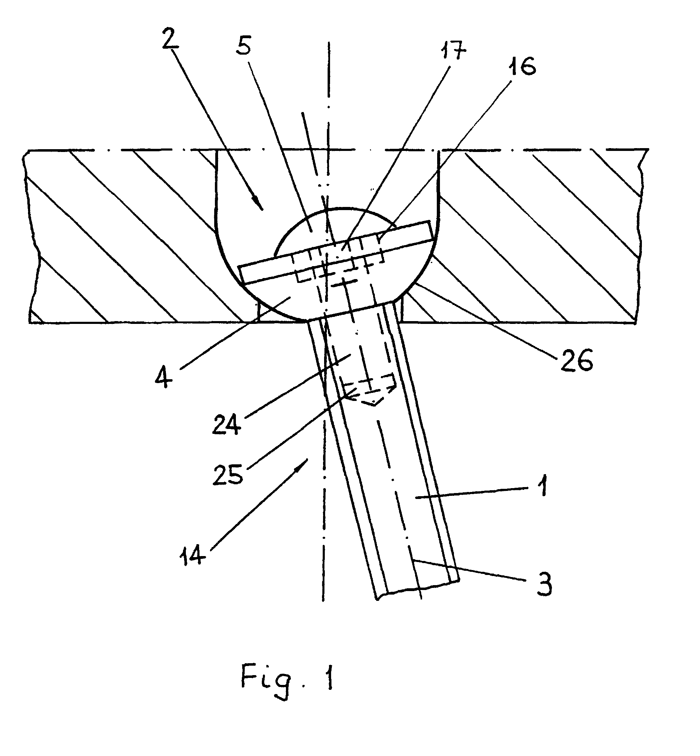

In Fig. 1 ist die bevorzugte Ausführungsform der erfindungsgemässen zweiteiligen Knochenschraube 14 dargestellt. Die Knochenschraube 14 umfasst konzentrisch zur Schraubenlängsachse 3 einen Schraubenschaft 1 und einen in axialer Richtung zweiteiligen Schraubenkopf 2, welcher aus einem schraubenschaftseitigen Unterteil 4 und einem endständigen Oberteil 5 besteht. Der Schraubenschaft 1 dient zur Verankerung der Knochenschraube in einem Knochen oder Knochenteil. Das Unterteil 4 ist schraubenschaftseitig konvex ausgebildet und weist eine kugelschichtförmige Auflagefläche 26 auf. Unterteil 4 und Schraubenschaft 1 sind einstückig. Die Verbindung zwischen Oberteil 5 und Schraubenschaft 4 ist eine Presssitzverbindung. Am Oberteil 5 ist konzentrisch zur Schraubenlängsachse 3 ein Zapfen 24 angebracht, welcher in einer zur Schraubenlängsachse 3 konzentrische Bohrung 25 im Schraubenschaft 1 einführbar ist. Zum Eindrehen des Schraubenschaftes 1 in den Knochen oder das Knochenteil ist am Unterteil 4 konzentrisch zur Längsachse 3 ein Innensechskant 16 angebracht, wobei die Schlüsselweite des Innensechskantes 16 so gewählt ist, dass die Seitenflächen 17 des Innensechskantes 16 den Zapfen 24 nicht berühren. Das Oberteil 5 ist axial einseitig sphärisch ausgeführt, so dass der zusammengesetzte Schraubenkopf 2 durch das Oberteil 5 endständig konvex ausgebildet ist.

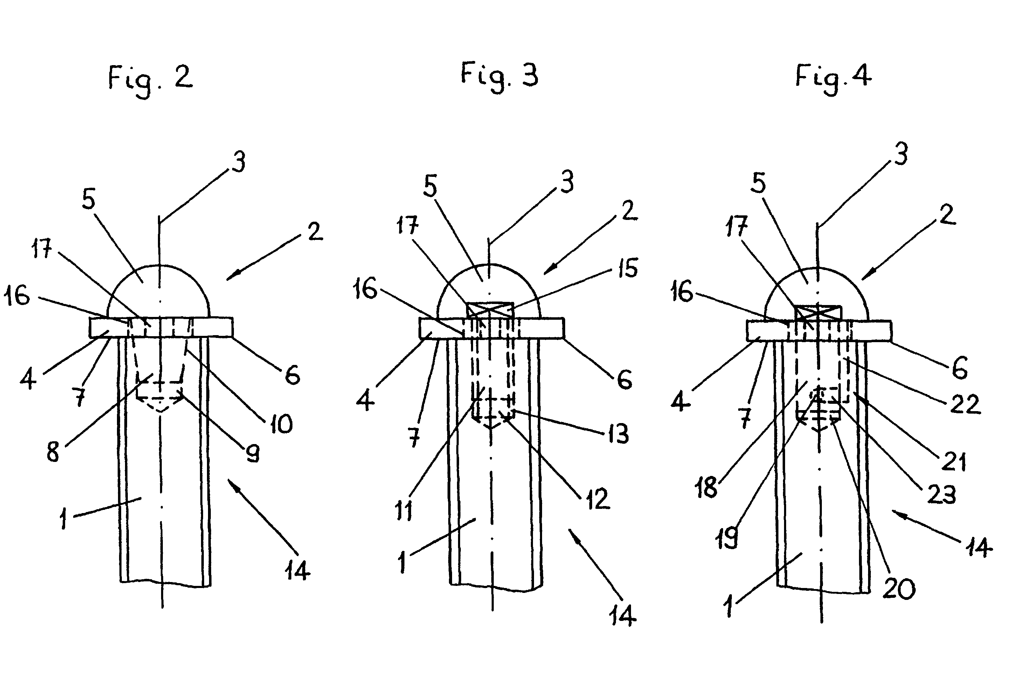

In Fig. 2 ist eine weitere Ausführungsform der ertindungsgemässen zweiteiligen Knochenschraube 14 dargestellt. Die Knochenschraube 14 umfasst konzentrisch zur Schraubenlängsachse 3 einen Schraubenschaft 1 und einen in axialer Richtung zweiteiligen Schraubenkopf 2, welcher aus einem schraubenschaftseitigen Unterteil 4 und einem endständigen Oberteil 5 besteht. Der Schraubenschaft 1 dient zur Verankerung der Knochenschraube in einem Knochen oder Knochenteil. Das Unterteil 4 ist kantig ausgebildet und weist schraubenschaftseitig eine ebene Auflagefläche 7 mit einer unteren Kante 6 auf. Unterteil 4 und Schraubenschaft 1 sind einstückig. Die Verbindung zwischen Oberteil 5 und Schraubenschaft 4 ist eine Konusverbindung. Am Oberteil 5 ist konzentrisch zur Schraubenlängsachse 3 ein konischer Zapfen 8 angebracht, welcher in einer zur Schraubenlängsachse 3 konzentrische Bohrung 9 mit einem Innenkonus 10 im Schraubenschaft 1 befestigbar ist. Zum Eindrehen des Schraubenschaftes 1 in den Knochen oder das Knochenteil ist am Unterteil 4 konzentrisch zur Längsachse 3 ein innensechskant 16 angebracht, wobei die Schlüsselweite des Innensechskantes 16 so gewählt ist, dass die Seitenflächen 17 des Innensechskantes 16 den konischen Zapfen 8 nicht berühren.

Fig. 3 zeigt eine weitere Ausführungsform der erfindungsgemässen zweiteiligen Knochenschraube 14, welche sich von der in Fig. 2 dargestellten Ausführungsform nur darin unterscheidet, dass die Verbindung zwischen Oberteil 5 und Schraubenschaft 1 eine Schraubverbindung ist. Am Oberteil 5 ist konzentrisch zur Schraubenlängsachse 3 ein Gewindezapfen 11 angebracht, welcher in eine zur Schraubenlängsachse 3 konzentrische Bohrung 12 mit einem Innengewinde 13 Schraubenschaft 1 schraubbar ist. Auch hier sind Unterteil 4 und Schraubenschaft 1 einstückig. Der Innensechskant 16 durchdringt axial das Unterteil 4, während die Bohrung 12 mit dem innengewinde 13 erst am unterteilseitigen Ende des Schraubenschaftes 1 beginnt und in diesen eindringt. Zudem sind am Oberteil 5 zwei oder mehr parallel zur Schraubenlängsachse 3 ausgerichtete Flächen 15 als Aussenzweikant zum Eindrehen des Schraubenschaftes 1 mittels eines geeigneten Schraubendrehers angebracht. Anstelle des Aussenzweikantes ist auch ein Aussenvierkant oder Aussensechskant möglich.

Fig. 4 zeigt wieder eine weitere Ausführungsform der erfindungsgemässen zweiteiligen Knochenschraube 14, welche sich von der in Fig. 3 dargestellten Ausführungsform nur darin unterscheidet, dass die Verbindung zwischen Oberteil 5 und Schraubenschaft 1 ein Bajonettverschluss ist. Am Oberteil 5 ist konzentrisch zur Schraubenlängsachse 3 ein Zapfen 18 mit einem radial vorstehenden Stift 19 angebracht, welcher in eine zur Schraubenlängsachse 3 konzentrische Bohrung 20 mit einer Nute 21 einschnappbar ist, wobei die Nute 21 einen parallel zur Schraubenlängsachse 3 verlaufenden Teil 22 und einer peripher in der Bohrung 20 verlaufenden Teil 23 aufweist.

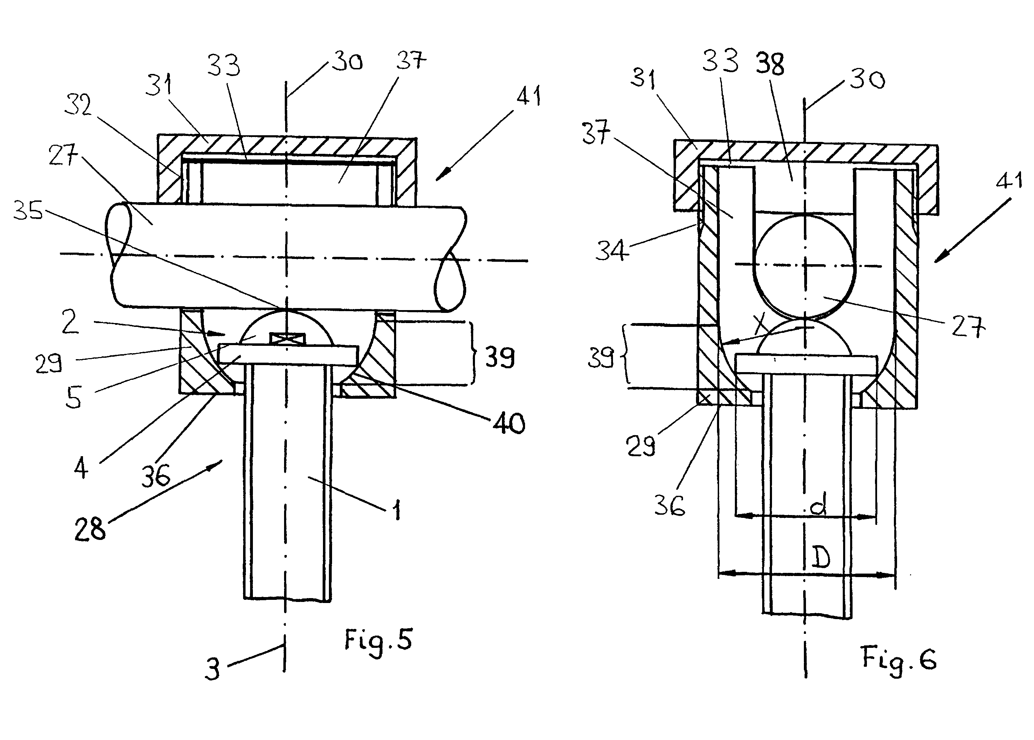

Die Fig. 5 und 6 zeigen eine Ausführungsform der erfindungsgemässen Vorrichtung, welche zur Verbindung eines Längsträgers 27 mit einer Pedikelschraube 28 innerhalb eines Wirbelsäulenfixationssystems dient. Diese Vorrichtung umfasst eine Pedikelschraube 28, welche konzentrisch zu ihrer Schraubenlängsachse 3 einen im Knochen zu verankemden Schraubenschaft 1 und einen Schraubenkopf 2 aufweist, einen Aufnahmekopf 29 mit der Zentralachse 30, welcher zur Verbindung eines Längsträgers 27 mit der Pedikelschraube 28 dient, und Spannmittel 31. Diese Spannmittel 31 weisen im wesentlich die Form einer Mutter auf, sind mittels eines Innengewindes 32 über ein an die schraubenkopfseitige Oberseite 33 des Aufnahmekopfes 29 angrenzendes Aussengewinde 34 schraubbar und dienen zur Fixierung des Längsträgers 27 und der Pedikelschraube 28 innerhalb des Aufnahmekopfes 29.

Der Schraubenkopf 2 ist zweitstückig und weist ein Unterteil 4 und ein Oberteil 5 auf, deren Verbindung nach einer der in den Fig. 1 bis 4 beschriebenen Ausführungsform ausgeführt ist. Das Oberteil 5 ist axial einseitig kugelsegmentförmig ausgeführt, wobei der Zenit 35 des Kugelsegmentes auf der Schraubenlängsachse 3 liegt und das schraubenkopfseitige Ende der Pedikelschraube 28 bildet.

Der Aufnahmekopf 29 weist eine schraubenkopfseitige Oberseite 33, eine schraubenschaftseitige Unterseite 36, eine koaxial zur Zentralachse 30 den Aufnahmekopf 29 durchdringende Durchgangsbohrung 37 zur Aufnahme der Pedikelschraube 28 und zusätzlich einen quer zur Zentralachse 30 verlaufenden gegen die Oberseite 33 offenen Kanal 38 zur Aufnahme eines Längsträgers 27 auf. Auf diese Weise ist der Längsträger 27 von der Oberseite 33 her in den offenen Kanal 38 einlegbar und dort mittels des Spannmittels 31 in lösbarer Weise fixierbar.

Die Durchgangsbohrung 37 umfasst einen konkaven sich gegen die Unterseite 36 verjüngenden Abschnitt 39, welcher in der hier dargestellten Ausführungsform der erfindungsgemässen Vorrichtung kugeischichtartig ausgestaltet ist.

Der zweistückige Schraubenkopf 2 weist ein konzentrisches kreisscheibenförmiges Unterteil 4 mit einem Durchmesser d auf. Der konkave Abschnitt 39 ist sphärisch mit einem Krümmungsradius X ausgebildet und mündet gegen die Oberseite 33 in einen zylindrischen Abschnitt mit dem Durchmesser D. In der hier gezeigten Ausführungsform der erfindungsgemässen Vorrichtung entspricht der Krümmungsradius X dem Radius des zylindrischen Abschnittes X = D/2. Ebenfalls ist in der hier gezeigten Ausführungsform der Durchmesser d des Unterteils 4 kleiner als der Durchmesser D des zylindrischen Abschnittes d < D. Diese Dimensionierung des Unterteils 4 und des konkaven Abschnittes 39 ermöglicht, dass das Unterteil 4 im konkaven Abschnitt 39 der Durchgangsbohrung 37 unter verschiedenen Winkeln zwischen Schraubenlängsachse 3 und Zentralachse 30 an der Wand 40 der Durchgangsbohrung 37 zur Anlage bringbar ist

## Patentansprüche

1. Knochenschraube (14;28) zur osteosynthetischen Knochenfixation mit einem zur Schraubenlängsachse (3) konzentrisch in einem Knochen oder Knochenteil zu verankernden Schraubenschaft (1) und einem Schraubenkopf (2), wobei
A) die Knochenschraube (14;28) axial mehrteilig ist;
B) mindestens der Schraubenkopf (2) in axialer Richtung zweistückig ist und ein schraubenschaftseitiges Unterteil (4) und ein endständiges, mit dem Unterteil (4) und/oder dem Schraubenschaft (1) verbindbares Oberteil (5) umfasst; und
C) das Oberteil (5) konvex ausgebildet ist,
**dadurch gekennzeichnet, dass**
D) das Unterteil (4) kreisscheibenförmig ausgebildet ist, wobei der Rand des Unterteils (4) kantig ausgebildet ist und eine untere Kante (6) aufweist.

2. Knochenschraube (14;28) nach Anspruch 1, **dadurch gekennzeichnet, dass** das Oberteil (5) mit dem Unterteil (4) und/oder dem Schraubenschaft (1) lösbar verbindbar ist.

3. Knochenschraube (14;28) nach Anspruch 1 oder 2, **dadurch gekennzeichnet, dass** das Unterteil (4) und der Schraubenschaft (1) einstückig sind.

4. Knochenschraube (14;28) nach Anspruch 2 oder 3, **dadurch gekennzeichnet, dass** das Oberteil (5) mittels einer Konusverbindung mit dem Schraubenschaft (1) verbindbar ist.

5. Knochenschraube (14;28) nach Anspruch 2 oder 3, **dadurch gekennzeichnet, dass** das Oberteil (5) mittets einer Schraubverbindung mit dem Schraubenschaft (1) verbindbar ist.

6. Knochenschraube (14;28) nach Anspruch 2 oder 3, **dadurch gekennzeichnet, dass** das Oberteil (5) mittels eines Bajonettverschlusses mit dem Schraubenschaft (1) verbindbar ist.

7. Knochenschraube (14;28) nach einem der Ansprüche 1 bis 6, **dadurch gekennzeichnet, dass** das Unterteil (4) schraubenschaftseitig konvex ausgebildet ist.

8. Knochenschraube (14;28) nach Anspruch 7, **dadurch gekennzeichnet, dass** das Unterteil (4) schraubenschaftseitig kugelschichtförmig ausgebildet ist.

9. Knochenschraube (14;28) nach einem der Ansprüche 1 bis 8, **dadurch gekennzeichnet, dass** das Oberteil (22) sphärisch ausgebildet ist.

10. Knochenschraube (14;28) nach Anspruch 9, **dadurch gekennzeichnet, dass** das Oberteil (5) halbkugelförmig ausgebildet ist.

11. Knochenschraube (14;28) nach einem der Ansprüche 1 bis 10, **dadurch gekennzeichnet, dass** der Durchmesser d des Unterteiles (4) zwischen 8-10 mm beträgt.

12. Knochenschraube (14;28) each Anspruch 1 bis 11, **dadurch gekennzeichnet, dass** das Unterteil (4) eine Dicke von 1-2 mm hat.

13. Knochenschraube (14;28) nach einem der Ansprüche 1 bis 12, **dadurch gekennzeichnet, dass** der Aussendurchmesser des Schraubenschaftes (1) 5-6 mm beträgt.

14. Knochenschraube (2) nach einem der Ansprüche 1 bis 13, **dadurch gekennzeichnet, dass** der Durchmesser d des Unterteiles (4) zwischen 4-6 mm beträgt.

15. Knochenschraube (2) nach Anspruch 14, **dadurch gekennzeichnet, dass** das Unterteil (4) eine Dicke von 0,5 - 1 mm hat.

16. Knochenschraube (2) nach Anspruch 14 oder 15, **dadurch gekennzeichnet, dass** der Aussendurchmesser des Schraubenschaftes (1 ) 3-5 mm beträgt.

17. Knochenschraube (14;28) nach einem der Ansprüche 1 bis 16, **dadurch gekennzeichnet, dass** sie zur Fixation von Knochen oder Knochenteilen innerhalb einer osteosynthetischen Fixationsvorrichtung dient.

18. Knochenschraube (14;28) nach Anspruch 17, **dadurch gekennzeichnet, dass** sie zur Fixation von Knochen oder Knochenteilen an einer Knochenplatte dient.

19. Knochenschraube (2) nach Anspruch 17, **dadurch gekennzeichnet, dass** sie eine Pedikelschraube (14;28) ist und zur Fixation von Wirbelkörpern innerhalb einer Wirbelsäuienfixationsvorrichtung dient.

20. Vorrichtung zur osteosynthetischen Knochenfixation mit mindestens einer Knochenschraube (14;28) gemäss einem der Ansprüche 1 bis 19,
**dadurch gekennzeichnet, dass**
A) sie zusätzliche mindestens einen plattenförmigen, prismatischen oder zylindrischen Fixationskörper (41) umfasst, welcher mindestens eine Bohrung (37) mit einer Zentralachse (30) zur Aufnahme der Knochenschraube (14;28), eine schraubenschaftseitige Unterseite (36) and sine schraubenkopfseitige Oberseite (33) aufweist, wobei
B) die Bohrung (37) einen konkaven sich gegen die Unterseite (36) verjüngenden Abschnitt (39) umfasst; und
C) der Durchmesser d des Unterteils (4) so dimensioniert ist, dass das Unterteil (4) im konkaven Abschnitt (39) der Bohrung (37) unter verschiedenen Winkeln zwischen Schraubenlängsachse (3) und Zentralachse (30) an der Wand (40) der Bohrung (37) zur Anlage bringbar ist.

21. Vorrichtung nach Anspruch 20, **dadurch gekennzeichnet, dass**
A) die Knochenschraube (14;28) eine Pedikelschraube (14;28) mit einem konvexen Schraubenkopf (2) ist;
B) der Fixationskörper (41) ein Aufnahmekopf (29) mit der Zentralachse (30) ist und zusätzlich einen quer zur Zentralachse (30) verlaufenden, gegen die Oberseite (33) offenen Kanal (38) zur Aufnahme eines Längsträgers (27) aufweist; und
C) die Vorrichtung zusätzlich Spannmittel (31) umfasst, welche von der Oberseite (33) her mit dem Aufnahmekopf (29) in ldsbar Weise verbindbar sind und zur Fixierung eines Längsträgers (27) und der Pedikelschraube (14;28) innerhalb des Aufnahmekopfes (29) dienen.

22. Vorrichtung nach Anspruch 20 oder 21, **dadurch gekennzeichnet, dass** das Unterteil (4) den Durchmesser d aufweist und der konkave Abschnitt (39) sphärisch ausgebildet ist und den Durchmesser D aufweist, wobei D = d ist.

23. Vorrichtung nach Anspruch 20 oder 21, **dadurch gekennzeichnet, dass** das Unterteil (4) den Durchmesser d aufweist und der konkave Abschnitt (39) sphärisch ausgebildet ist und den Durchmesser D aufweist, wobei D > d ist.

24. Vorrichtung nach Anspruch 23, **dadurch gekennzeichnet, dass** das Verhäitnis d : D zwischen 0,5 und 1,0 liegt.

25. Vorrichtung nach Anspruch 24, **dadurch gekennzeichnet, dass** das Verhältnis d : D zwischen 0,85 und 0,95 liegt.

26. Vorrichtung nach einem der Ansprüche 23 bis 25, **dadurch gekennzeichnet, dass** der konkave Abschnitt (39) kugelschichtartig ausgebildet ist, wobei die Kugelschicht einen Radius X aufweist und X ≥ D/2 ist.

27. Vorrichtung nach Anspruch 26, **dadurch gekennzeichnet, dass** das Verhältnis von D/2 zu X zwischen 0,5 und 1,0 beträgt.

28. Vorrichtung nach Anspruch 27, **dadurch gekennzeichnet, dass** das Verhältnis von D/2 zu X zwischen 0,85 und 0,95 beträgt.

29. Vorrichtung nach einem der Ansprüche 20 bis 28, **dadurch gekennzeichnet, dass** der Durchmesser D des konkaven Abschnittes (39) und der Durchmesser d des Unterteils (4) zwischen 8-10 mm betragen.

30. Vorrichtung nach einem der Ansprüche 20 bis 29, **dadurch gekennzeichnet, dass** der Fixationskörper (41) eine Knochenplatte mit mindestens einer durchgehenden Bohrung für eine Knochenschraube (14) ist.

## Claims

1. A bone screw (14; 28) for osteosynthetic bone fixation, with a screw shank (1) to be anchored concentric to the longitudinal axis (3) of the screw in a bone or bone part, and with a screw head (2), whereby
A) the bone screw (14; 28) is in more than one part axially,
B) at least the screw head (2) being in two parts in the axial direction and comprising a bottom part (4) toward the screw shank and a top part (5) toward the end which can be connected to the bottom part (4) and/or to the screw shank (1); and
C) the upper part (5) being of convex design;
**characterized in that**
D) the bottom part (4) is designed as a circular disk, whereby the rim of the bottom part (4) is stepped and has a lower edge (6).

2. The bone screw (14; 28) as claimed in claim 1, **characterized in that** the top part (5) can be connected in a releasable manner to the bottom part (4) and/or to the screw shank (1).

3. The bone screw (14; 28) as claimed in claim 1 or 2, **characterized in that** the bottom part (4) and the screw shank (1) are in one piece.

4. The bone screw (14; 28) as claimed in claim 2 or 3, **characterized in that** the top part (5) can be connected to the screw shank (1) by means of a cone connection.

5. The bone screw (14; 28) as claimed in claim 2 or 3, **characterized in that** the top part (5) can be connected to the screw shank (1) by means of a screw connection.

6. The bone screw (14; 28) as claimed in claim 2 or 3, **characterized in that** the top part (5) can be connected to the screw shank (1) by means of a bayonet lock.

7. The bone screw (14; 28) as claimed in one of claims 1 through 6, **characterized in that** the bottom part (4) is of convex design toward the screw shank.

8. The bone screw (14; 28) as claimed in claim 7, **characterized in that** the bottom part (4) is designed as a segment of a sphere toward the screw shank.

9. The bone screw (14; 28) as claimed in one of claims 1 through 8, **characterized in that** the top part (22) is of spherical design.

10. The bone screw (14; 28) as claimed in claim 9, **characterized in that** the top part (5) is of semispherical design.

11. The bone screw (14; 28) as claimed in one of claims 1 through 10, **characterized in that** the diameter d of the bottom part (4) is between 8 and 10 mm.

12. The bone screw (14; 28) as claimed in claims 1 through 11, **characterized in that** the bottom part (4) has a thickness of 1 to 2 mm.

13. The bone screw (14; 28) as claimed in one of claims 1 through 12, **characterized in that** the external diameter of the screw shank (1) is 5 to 6 mm.

14. The bone screw (2) as claimed in one of claims 1 through 13, **characterized in that** the diameter d of the bottom part (4) is between 4 and 6 mm.

15. The bone screw (2) as claimed in claim 14, **characterized in that** the bottom part (4) has a thickness of 0.5 to 1 mm.

16. The bone screw (2) as claimed in claim 14 or 15, **characterized in that** the external diameter of the screw shank (1) is 3 to 5 mm.

17. The bone screw (14;28) as claimed in one of claims 1 through 16, **characterized in that** it serves for the fixation of bones or bone parts in an osteosynthesis fixation device.

18. The bone screw (14; 28) as claimed in claim 17, **characterized in that** it serves for the fixation of bones or bone parts on a bone plate.

19. The bone screw (2) as claimed in claim 17, **characterized in that** it is a pedicle screw (14; 28) and serves for the fixation of vertebrae in a spinal column fixation device.

20. A device for osteosynthetic bone fixation with at least one bone screw (14; 28) as claimed in one of claims 1 through 19, **characterized in that**
A) it additionally comprises at least one plate-shaped, prismatic or cylindrical fixation body (41) which has at least one bore (37) with a central axis (30) for receiving the bone screw (14; 28), an underside (36) toward the screw shank, and an upper side (33) toward the screw head,
B) the bore (37) comprising a concave portion (39) tapering toward the underside (36), and
C) the diameter d of the bottom part (4) being dimensioned such that the bottom part (4), in the concave portion (39) of the bore (37), can be made to bear on the wall (40) of the bore (37) at different angles between the longitudinal axis (3) of the screw and the central axis (30).

21. The device as claimed in claim 20, **characterized in that**
A) the bone screw (14; 28) is a pedicle screw (14; 28) with a convex screw head (2);
B) the fixation body (41) is a receiving head (29) with central axis (30), and additionally has a channel (38) extending transverse to the central axis (30) and open toward the upper side (33) in order to receive a longitudinal support (27); and
C) the device additionally comprises clamping means (31) which can be connected to the receiving head (29) in a releasable manner from the direction of the upper side (33) and serve for fixing a longitudinal support (27) and the pedicle screw (14; 28) within the receiving head (29).

22. The device as claimed in claim 20 or 21, **characterized in that** the bottom part (4) has the diameter d, and the concave portion (39) is of spherical design and has the diameter D, where D = d.

23. The device as claimed in claim 20 or 21, **characterized in that** the bottom part (4) has the diameter d, and the concave portion (39) is of spherical design and has the diameter D, where D > d.

24. The device as claimed in claim 23, **characterized in that** the ratio d : D is between 0.5 and 1.0.

25. The device as claimed in claim 24, **characterized in that** the ratio d : D is between 0.85 and 0.95.

26. The device as claimed in one of claims 23 through 25, **characterized in that** the concave portion (39) is designed as a segment of a sphere, where the spherical segment has a radius X, and X ≥ D/2.

27. The device as claimed in claim 26, **characterized in that** the ratio of D/2 to X is between 0.5 and 1.0.

28. The device as claimed in claim 27, **characterized in that** the ratio of D/2 to X is between 0.85 and 0.95.

29. The device as claimed in one of claims 20 through 28, **characterized in that** the diameter D of the concave portion (39) and the diameter d of the bottom part (4) are between 8 and 10 mm.

30. The device as claimed in one of claims 20 through 29, **characterized in that** the fixation body (41) is a bone plate with at least one through-bore for a bone screw (14).

## Revendications

1. Vis à os (14; 28) pour la fixation osseuse en vue d'ostéosynthése, avec une tige de vis (1) à ancrer concentriquement par rapport à l'axe longitudinal (3) de la vis dans un os ou une partie d'os, et une tête de vis (2),
A) la vis à os (14; 28) étant réalisée en plusieurs piéces dans le sens axial;
B) au moins la tête de vis (2) est réalisée en deux pièces dans la direction axiale et comporte une partie inférieure (4) située du côté de la tige de la vis et une partie supérieure (5) située du côté de l'extrémité et apte à être reliée à la partie inférieure (4) et/ou à la tige (1) de la vis; et
C) la partie supérieure (5) présente une configuration convexe aisée en ce que
D) la partie inférieure (4) est configurée en forme de plaque circulaire, le bord de la partie inférieure (4) étant chanfreiné et présentant une arête inférieure (6).

2. Vis à os (14; 28) selon la revendication 1, **caractérisée en ce que** la partie supérieure (5) peut être reliée de manière libérable à la partie inférieure (4) et/ou à la tige (1) de la vis.

3. Vis à os (14; 28) selon la revendication 1 ou 2, **caractérisée en ce que** la partie inférieure (4) et la tige (1) de la vis sont réalisées d'un seul tenant.

4. Vis à os (14; 28) selon la revendication 2 ou 3, **caractérisée en ce que** la partie supérieure (5) peut être reliée à la tige (1) de la vis au moyen d'une liaison conique.

5. Vis à os (14; 28) selon la revendication 2 ou 3, **caractérisée en ce que** la partie supérieure (5) peut être reliée à la tige (1) de la vis au moyen d'une liaison vissée.

6. Vis à os (14; 28) selon la revendication 2 ou 3, **caractérisée en ce que** la partie supérieure (5) peut être reliée à la tige (1) de la vis au moyen d'une fermeture à baïonnette.

7. Vis à os (14; 28) selon l'une des revendications 1 à 6, **caractérisée en ce que** la partie inférieure (4) présente une configuration convexe du côté de la tige de vis.

8. Vis à os (14; 28) selon la revendication 7, **caractérisée en ce que** la partie inférieure (4) présente une configuration en calotte sphérique du côté de la tige de la vis.

9. Vis à os (14; 28) selon l'une des revendications 1 à 8, **caractérisée en ce que** la partie supérieure (22) présente une configuration sphérique.

10. Vis à os (14; 28) selon la revendication 9, **caractérisée en ce que** la partie supérieure (5) présente une configuration hémisphérique.

11. Vis à os (14; 28) selon l'une des revendications 1 à 10, **caractérisée en ce que** le diamétre d de la partie inférieure (4) est compris entre 8 et 10 mm.

12. Vis à os (14; 28) selon les revendications 1 à 11, **caractérisée en ce que** la partie inférieure (4) présente une épaisseur de 1 à 2 mm.

13. Vis à os (14; 28) selon l'une des revendications 1 à 12, **caractérisée en ce que** le diamètre extérieur de la tige (1) de la vis est compris entre 5 et 6 mm.

14. Vis à os (2) selon l'une des revendications 1 à 13, **caractérisée en ce que** le diamètre d de la partie inférieure (4) est compris entre 4 et 6 mm.

15. Vis à os (2) selon la revendication 14, **caractérisée en ce que** la partie inférieure (4) représente une épaisseur comprise entre 0,5 et 1 mm.

16. Vis à os (2) selon la revendication 14 ou 15, **caractérisée en ce que** le diamètre extérieur de la tige (1) de la vis est compris entre 3 et 5 mm.

17. Vis à os (14; 28) selon l'une des revendications 1 à 16, **caractérisée en ce qu'**elle sert à la fixation d'os ou de parties d'os dans un dispositif de fixation pour ostéosynthèse.

18. Vis à os (14; 28) selon la revendication 17, **caractérisée en ce qu'**elle sert à la fixation d'os ou de parties d'os sur une plaque à os.

19. Vis à os (2) selon la revendication 17, **caractérisée en ce qu'**elle est une vis à pédicule (14; 28) et qu'elle sert à la fixation de corps de vertèbres dans un dispositif de fixation de la colonne vertébrale.

20. Dispositif pour la fixation d'os en vue d'ostéosynthèse, comportant au moins une vis à os (14; 28) selon l'une des revendications 1 à 19, **caractérisé en ce que** :
A) il comprend en plus au moins un corps de fixation (41) en forme de plaque prismatique ou cylindrique, qui présente au moins un alésage (37) doté d'un axe central (30) pour la réception de la vis à os (14; 28), une partie inférieure (36) située du côté de la tige de vis et une partie supérieure (33) située du côté de la tête de vis,
B) l'alésage (37) comprenant une partie (39) concave qui se rétrécit en direction du côté inférieur (36), et
C) le diamètre d de la partie inférieure (4) étant dimensionné de telle sorte que la partie inférieure (4) puisse être amenée à se placer dans la partie concave (39) de l'alésage (37) contre la paroi (40) de l'alésage (37) sous différents angles entre l'axe longitudinal (3) de la vis et l'axe central (30).

21. Dispositif selon la revendication 20, **caractérisé en ce que**
A) la vis à os (14; 28) est une vis à pédicule (14; 28) dotée d'une tête de vis (2) convexe;
B) le corps de fixation (41) est une tête de réception (29) dotée d'un axe central (30) et présente de plus un canal (38) qui s'étend transversalement par rapport à l'axe central (30) et ouvert en direction du côté inférieur (33), pour la réception d'un support longitudinal (27), et
C) le dispositif comprend des moyens supplémentaires de serrage (31) qui peuvent être reliés de manière libérable à la tête de réception (29) par le côté supérieur (33) et qui servent à fixer un support longitudinal (27) et la vis à pédicule (14; 28) dans la tête de réception (29).

22. Dispositif selon la revendication 20 ou 21, **caractérisé en ce que** la partie inférieure (14) présente le diamètre d et **en ce que** la partie concave (39) présente une configuration sphérique et le diamètre D, D = d.

23. Dispositif selon la revendication 20 ou 21, **caractérisé en ce que** la partie inférieure (4) présente le diamètre d et **en ce que** la partie concave (39) présente une configuration sphérique et le diamètre D, D > d.

24. Dispositif selon la revendication 23, **caractérisé en ce que** le rapport d : D est compris entre 0,5 et 1,0.

25. Dispositif selon la revendication 24, **caractérisé en ce que** le rapport d : D est compris entre 0,85 et 0,95.

26. Dispositif selon l'une des revendications 23 à 25, **caractérisé en ce que** la partie concave (39) est configurée en forme de calotte sphérique, la calotte sphérique présentant un rayon X et X ≥ D/2.

27. Dispositif selon la revendication 26, **caractérisé en ce que** le rapport entre D/2 et X est compris entre 0,5 et 1,0.

28. Dispositif seion la revendication 27, **caractérisé en ce que** le rapport entre D/2 et X est compris entre 0,85 et 0,95.

29. Dispositif selon l'une des revendications 20 à 28, **caractérisé en ce que** le diamètre D de la partie concave (39) et le diamètre d de la partie inférieure (4) sont compris entre 8 et 10 mm.

30. Dispositif selon l'une des revendication 20 à 29, **caractérisé en ce que** le corps de fixation (41) est une plaque pour os traversée par au moins un alésage pour une vis à os (14).
